# EUROPEAN PATENT SPECIFICATION

(11) **EP 1 042 268 B1**
(45) Date of publication and mention of the grant of the patent: **07.05.2003**
(21) Application number: 98957044.5
(22) Date of filing: 26.11.1998
(51) Int. Cl.: C07C 49/697, C07C 65/36, C07C 255/56, C07C 233/78, C07C 257/18

(54) **1,7-DIBROMODIBENZOSUBERONE AND PROCEDURES FOR ITS PREPARATION**
1,7-DIBROMODIBENZOSUBERON UND VERFAHREN ZUR HERSTELLUNG
1,7-DIBROMODIBENZOSUBERONE ET PROCEDES DE PREPARATION

(30) Priority: 01.12.1997 HR 970649
(43) Date of publication of application: 11.10.2000
(60) Divisional application: 03002060.6
(73) Proprietor: Belupo - Lijekovi I Kozmetika D.O.O., 48000 Koprivnica (HR)
(72) Inventor: MIKOTIC-MIHUN, Zvonimitra, HR-10000 Zagreb (HR); KARMINSKI-ZAMOLA, Grace, HR-10000 Zagreb (HR); CEPANEC, Ivica, HR-42204 Turcin (HR); LITVIC, Mladen, HR-48362 Virje (HR)
(74) Representative: Rupprecht, Kay, Dipl.-Ing.
(86) International application number: HR9800008
(87) International publication number: WO99028284

(56) References cited:
- CH-A- 541 545
- HOLM T: "Preparation and properties of 1,1-diaryl-1,3-butadienes" ACTA CHEMICA SCANDINAVICA., vol. 17, 1963, pages 2437-2442, XP002096402 COPENHAGEN DK
- WALKER G ET AL: "Synthesis of 10,11-dihydro-5,10-ethano-5H-dibenzo[a,d]c ycloheptenes with various side chains at position 12" J. ORG. CHEM. (JOCEAH);1972; VOL.37 (26); PP.4294, XP002096403 Ciba-Geigy Corp.;Pharm. Div.; Summit; N. J.
- DUNN J P ET AL: "Dibenzotropone- and dibenzosuberonecarboxylic acids with bronchodilator activity" J. MED. CHEM. (JMCMAR,00222623);1979; VOL.22 (11); PP.1357-63, XP002096404 Inst. Org. Chem.;Palo Alto; 94304; CA; USA

## Description

The invention is referred to the following chemical compound: 1,7 dibromodibenzosuberone (1) and the procedures of its preparation. The most related known compounds are 8-substituted derivatives of dibenzosuberone-3-yl-acetic acid, prepared by the total synthesis from simple phenylic derivatives [Germ. Pat. 2,224,655 (1972)]. The preparation of 3-bromodibenzosuberone (11) by the total synthesis from p-bromophenylacetic acid and phthalaldehyde in a three-step reaction, as well as some other alternative synthetic procedures have also been described in the scientific literature [Ebnother et al., Helv. Chim. Acta, 48 (6) (1965) 1237; Bastian et al., Helv. Chim. Acta, 49 (1966) 214]. Preparations of variously monosubstituted dibenzosuberone derivatives have been described as well [Hrnciar, Helv. Chem. Zvesti, XVI 1-2 (1962) 96]. 3,7-dibromodibenzosuberone (2) has been described in Acta Chemica Scandinavica, 17, 1963, 2437-2442. The subject of this invention is the bromine derivative of dibenzosuberone 1 with general formula I, where radicals R1 - R6 represent hydrogen or bromine, as well as the procedures of its preparation by the methods of direct bromination of the available dibenzosuberone, and the effective procedures of its isolation. 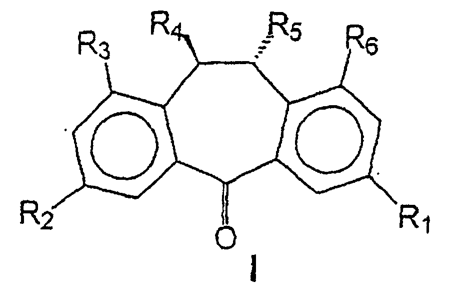
1: R₂, R₄, R₅, R₆ = H, R₁, R₃ = Br
2: R₃, R₄, R₅, R₆ = H, R₁, R₂ = Br

1,7-dibromodibenzosuberone (1) was prepared according to four different procedures. By the bromination of dibenzosuberone with 2 - 4 of bromine equivalents in 1,2-dichloroethane, dichloromethane, tetrachloromethane or chloroform with:
1. Aluminum chloride as a Lewis' acid, with optimal quantity of 1 - 8 equivalents in relation to dibenzosuberone.
2. Aluminum chloride along with nitrobenzene, with optimal quantity of 1- 6 equivalents of aluminum chloride in relation to dibenzosuberone. The optimal ratio aluminum chloride/nitrobenzene should be 10:1 to 1:4.
3. Aluminum chloride along with triethanolamine, with optimal quantity of 1 - 6 equivalents of aluminum chloride in relation to dibenzosuberone, and with aluminum chloride/triethanolamine ratio between 10:11 and 1:1.
4. Aluminum chloride along with tris(hydroxymethyl)-aminomethane, with optimal quantity of 1 - 6 equivalents of aluminum chloride in relation to dibenzosuberone, and with aluminum chloride/tris (hydroxymethyl) aminomethane ratio between 2:1 and 8:1. Reaction concentrations of dibenzosuberone are between 10 and 140g 45 in 1000 ml of above quoted reaction solvents. The reactions are performed by cooling (-5° C to +5° C) of dibenzosuberone solution and aluminum chloride/nitrobenzene, or aluminum chloride/triethanolamine, or aluminum chloride/tris (hydroxymethyl)-aminomethane in 1,2-dichloroethane, dichloro-methane, chloroform or tetrachloromethane, and by adding dropwise bromine diluted in the same solvent, in a dry atmosphere. The reaction lasts for 20 - 94 hours at the temperature between - 5° C and room temperature. After the reaction is completed, the reaction mixture is treated with the ice-cold solution of sodium sulfite or bisulfite, and is extracted (dichloro- 5 methane, chloroform, tetrachloromethane or 1,2-dichloroethane), the extracts are dried over sodium or magnesium sulfate, and are evaporated. A small amount of ethyl acetate is added to the residue and the crystallization of the compound 2 takes place; it is aspirated, rinsed with petrolether or lower alkanes, and dried. The pooled mother solutions are evaporated, and 1,7-dibromo-dibenzosuberone (1) is obtained following the evaporation of fractions with R_{f} = 0.50 (TLC), while 1,9-dibromodibenzosuberone is obtained by evaporation of fractions with R_{f} = 0.45 (TLC) by a chromatographic separation on the silica gel column with dichloromethane/n-hexane (1:1) as eluent. Crude products are purified by recrystallization from ethyl acetate or from other lower esters.

According to this invention, bromine derivatives of dibenzosuberone can be prepared by a direct regioselective bromination of available dibenzosuberone, and by the adequate procedures of isolation.

### Reference Example 1

### 3,7-Dibromodibenzosuberone (2) along with aluminum chloride

Add dropwise the bromine solution (2.25 ml, 0.044 mol) in 1,2-dichloroethane (20 ml) into the cold (0°C) solution of dibenzosuberone (4.16 g, 40 0.02 mol) and aluminum chloride (11.73 g, 0.088 mol), and mix for 24 hours in a dry atmosphere. Then pour into the water (100 ml) containing some ice and dissolved sodium sulfite (2g) and hydrochloric acid (10 ml). Mix the reaction mixture for 1 hour, then separate organic layer, and extract the aqueous layer with dichloromethane (3 x 50 ml). Dry the pooled organic extracts over sodium sulfate, filter and evaporate off. Crystalize the residue from ethyl acetate. 0.73 g (10.1%) of dibromide 2 (colorless crystals) is obtained. M.p. 145.5-147° C.
IR (KBr) v: 3080, 3040, 2935, 2920, 2850, 1638 (C=O), 1572, 1563, 1440, 1425, 1390, 1345, 1285, 1255, 1227, 1205, 1143, 1120, 1090, 1080, 984, 953, 923, 914, 900, 825, 815, 807, 788, 776, 714, 695, 676, 630.
¹H-NMR (CDCl₃)δ: 3.13 (s, 4H, CH₂, benzyli), 7.11 (d, 2H, arom., J=8.1), 7.53 (dd, 2H, arom., J=8.2), 8.11 (d, 2H, arom., J=2.2).
¹³C-NMR (CDCl₃)δ: 33.98, 120.63, 131.15, 133.39, 135.38, 139.35, 140.67, 192.34 (C= O).

| Elementary analysis: C₁₅H₁₀Br₂O (366.06) | | | |
|---|---|---|---|
| | C | H | Br |
| Calculated | 49.22% | 2.75% | 43.66% |
| Found | 49.20% | 3.04% | 43.61% |

### Reference Example 2

3,7-dibromodibenzosuberone (2) along with aluminum chloride/nitrobenzene cool the solution of dibenzosuberone (20.83 g, 0.1 mol), nitrobenzene (11.3 ml, 0.11 mol) and aluminum chloride (58.7 g, 0.44 mol) in 1,2- dichloroethane (200 ml), to - 5° C and add dropwise, the bromine solution (12.4 ml, 0.24 mol) in 1,2-dichloroethane (100 ml). After 24 hours pour the reaction mixture into the mixture of the ice (100 g), sodium sulfite solution in water (100 ml 10%) and conc. hydrochloric acid (50 ml), mix for 1 hour at room temperature, then separate the organic layer, and extract the aqueous layer with dichloromethane (3 x 100 ml). Dry the pooled organic extracts over sodium sulfate, filter and evaporate off. Heat the residue at 64 - 65° C and distil off nitrobenzene under the pressure of 0.7 m bar (0.55 mm Hg). The product crystallise in 12 hours from ethyl acetate (30 ml) in the form of yellowish crystals. After, 35 crystallization from ethyl acetate, 5.49 g (15%) a pure dibromide 2 is obtained. TLC (silica gel, with dlchloromethane/n-hexane as an eluent): R_{f} = 0.57. Spectroscopic particulars are the same as in Example 2.

### Reference Example 3

### 3,7- Dibromodibenzosuberone (2) along with aluminum chloride / triethanolamine

Add the solution of triethanolamine (1.49 g, 0.01 mol) in 1,2-dichloroethane (20 ml) into dibenzosuberone solution (4.163 g, 0.02 mol) in 1,2-dichloroethane (20 ml). To the reaction mixture cooled to - 5° C add aluminum chloride (11.73 g, 0.088 mol) and the bromine solution (2.27 ml, 0.44 ml) in 1,2-dichloroethane (20 ml). Mix the reaction mixture in a dry atmosphere at room temperature for 22 hours, then pour into the mixture of the ice (50 g), conc. hydrochloric acid (50 ml) and sodium sulfite (2 g). Separate the organic layer, and extract the aqueous layer with dichloromethane (3 x 50 ml). Dry the pooled organic extracts over sodium sulfate, filter and evaporate off. After recrystallization of the obtained product from ethyl acetate, 820 mg (11.2%) a pure dibromide 2, is obtained. Spectroscopic particulars are the same as in Example 2.

### Reference Example 4

### 3,7-Dibromodibenzosuberone (2) along with aluminum chloride/tris (hydroxymethyl) aminomethane

Add aluminum chloride (40 g, 0.3 mol) and tris (hydroxymethyl) aminomethane (6.052 g, 0.05 mol) to a dibenzosuberone solution (20.82 g, 0.1 mol) in 1,2-dichloroethane (200 ml) cooled to -5° C; after 30 minutes of mixing add the bromine solution (11.4 ml, 0.2 mol) in 1,2-dichloroethane (100 ml). Mix for 23 hours at room temperature, and then pour into the mixture of the ice (200 g), conc. hydrochloric acid (150 ml) and sodium sulfite (5 g), while constantly stirring. Separate the organic layer, and extract the aqueous layer with dichloromethane (3 x 100 ml). Dry the pooled organic extracts over sodium sulfate, filter and evaporate off. By recrystallization from ethyl acetate (20 ml) 7.76 g (21.2%) of dibromide 2 is obtained, with 99.9% of purity according to HPLC performed on LC-Si column (Supelco) with n-hexane/diethyl ether (95:5) + 0.15% of acetonitrile as an eluent (19 bars, room temperature). Spectroscopic particulars are the same as in Example 2.

### Example 1

### 1, 7-Dibromodibenzosuberone (1) along with aluminum chloride

After the aspiration of crystals from ethyl acetate (Example 2 and Example 4), pool the mother solutions and evaporate. A dense, brown oil is obtained (28.01 g) that is crystallized if left to stand. Perform the chromatography on a silica gel column with dichloromethane/n-hexane (1:1) as an eluent. Seventeen grams of 1,7-dibromo-dibenzosuberone (1) and 2.1 g of 1,9-dibromo-dibenzosuberone may be separated chromatographically.
1, 7-Dibromodibenzosuberone (1)
M. p. 87-88°C.
IR (KBr) v: 2950, 2930, 2910, 1658 (C=O), 1577, 1557, 1473, 1446, 1433, 1415, 1383, 1343, 1322, 1286, 1266, 1195, 1153, 1126, 1090, 1027, 965, 955, 940, 905, 876, 812, 800, 760, 725, 720, 680, 643, 630.
¹H-NMR (CDCl₃)δ: 3.11 (d, 1H, benzyl, J=2.1), 3.13 (d, 1H, benzyl, J=5.6), 3.29 (d, 1H, benzyl, J=5.4), 3.31 (d, 1H, benzyl, J=2.1), 7.07 - 7.18 (m, 2H, aromates), 7.49 - 7.52 (m, 1H, aromates), 7.67 - 7.76 (m, 2H, aromates), 7.95 (s, 1H, aromate).
¹³C-NMR (CDCl₃)δ: 32.66, 33.69, 120.50, 124.71, 127.88, 129.23, 131.06, 132.26, 135.12, 136.66, 139.78, 139.89, 139.98, 140.83, 194.72 (C=O).

| Elementary analysis: C₁₅H₁₀Br₂O (366.06) | | | |
|---|---|---|---|
| | C | H | Br |
| Calculated | 49.22% | 2.75% | 43.66% |
| Found | 49.25% | 2.92% | 43.70% |

## Claims

1. Dibenzosuberone derivative of general formula I, **characterized by** the fact that the radicals R₁, R₃, R₄ and R₅ represent hydrogens, while R₂ and R₆ are bromine radicals. 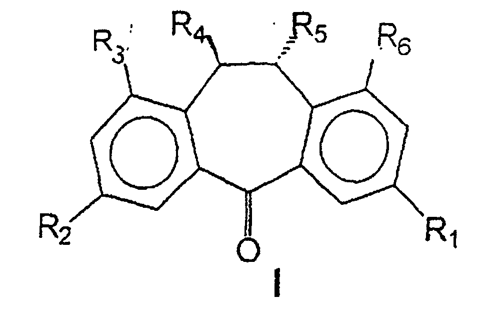
1: R₁, R₃, R₄, R₅ = H, R₂, R₆ = Br
2: R₃, R₄, R₅, R₆ = H, R₁, R₂ = Br

2. Preparation procedure for 1,7-dibromo-dibenzosuberone (1), pursuant to the Claim 1 general formula I, where radicals R1, R₃, R₄ and R₅ represent hydrogens, while R₂ and R₆ are bromine radicals, **characterized by** the fact that the compound 1 is obtained by a chromatographic separation on a silicia gel column with dichloromethane/n-hexan (1:1) as eluent and a R_{f}-Value of 0,50 (TLC) from the mother solution, following crystallization of the compound 2.

## Patentansprüche

1. Dibenzosuberonderivat der allgemeinen Formel I, **gekennzeichnet durch** die Tatsache, dass die Radikalen R₁, R₃, R₄ und R₅ Wasserstoffe, während R₂ und R₆ Bromradikale sind. 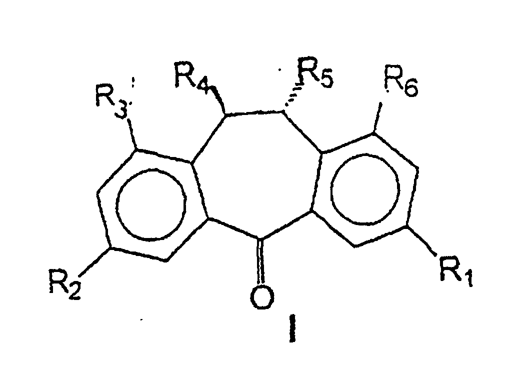
1: R₁, R₃, R₄, R₅ = H, R₂, R⁶ = Br
2: R₃, R₄, R₅, R₆ = H, R₁, R₂ = Br

2. Herstellungsverfahren für 1,7-Dibrom-Dibenzosuberon (1) nach Anspruch 1, allgemeine Formel I, bei dem die Radikalen R₁, R₃, R₄ und R₅ Wasserstoffe, während R₂ und R₆ Bromradikale sind, **gekennzeichnet durch** die Tatsache, dass die Verbindung 1 **durch** eine chromatographische Trennung auf einer Silicagel-Kolonne mit Dichlormethan/n-Hexan (1:1) als Eluanten und einem R_{f}-Wert von 0,50 (TLC) aus der Mutterlösung nach der Kristallisierung der Verbindung 2 erhalten wird.

## Revendications

1. Dérivé de dibenzosuberone de formule générale I, **caractérisé par le fait que** les radicaux R₁, R₃, R₄ et R₅ représentent des atomes d'hydrogène, tandis que R₂ et R₆ sont des radicaux brome. 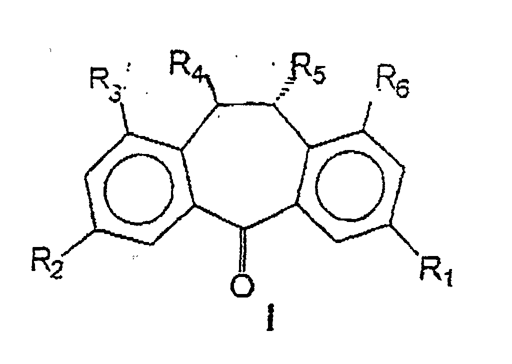
1: R₁, R₃, R₄, R₅ = H, R₂, R₆ = Br
2: R₃, R₄, R₅, R₆ = H, R₁, R₂ = Br

2. Procédé de préparation du 1,7-dibromo-dibenzosuberone (1), selon la formule générale I de la revendication 1, dans laquelle les radicaux R1, R₃, R₄ et R₅ représentent des atomes d'hydrogène, tandis que R₂ et R₆ sont des radicaux brome, **caractérisé par le fait que** le composé 1 est obtenu par séparation chromatographique sur une colonne de gel de silice avec, à titre d'éluant, un mélange dichlorométhane/n-hexane et une valeur R_{f} de 0,50 (TLC) à partir de la solution mère, suivi par une cristallisation du composé 2.
